# EUROPEAN PATENT APPLICATION

(11) **EP 3 438 257 A1**
(43) Date of publication of application: **06.02.2019**
(21) Application number: 17773668.3
(22) Date of filing: 06.02.2017
(51) Int. Cl.: C12N 15/09, C12Q 1/68, G06F 19/20

(54) **METHOD FOR DESIGNING MUTANT PRIMER**

(30) Priority: 30.03.2016 JP 2016067252; 04.08.2016 JP 2016154013
(71) Applicant: Daiken Medical Co., Ltd., Osaka-shi Osaka 5410045 (JP)
(72) Inventor: TAKAISHI, Makoto, Kawasaki-shi Kanagawa 213-0012 (JP)
(74) Representative: dompatent von Kreisler Selting Werner - Partnerschaft von Patent- und Rechtsanwälten mbB
(86) International application number: PCT/JP2017/004162
(87) International publication number: WO 2017/169119

(57) **Abstract**

Provided is a novel method for designing a mutant primer in which a nonspecific amplification caused by a primer dimer or loop structure scarcely occurs. A method for designing a primer having a mutation introduced thereinto, said method comprising a mutation introduction site-selection step for selecting, as a mutation introduction site, one or more nucleotide residues, said nucleotide residue(s) being contained in a basic primer sequence and satisfying one or more requirements selected from the group consisting of the following requirements (1) to (4): (1) a nucleotide residue possibly contributing to the formation of a primer dimer; (2) a nucleotide residue possibly contributing to the formation of a loop structure in a single primer molecule; (3) when it is predicted that a primer comprising the aforesaid basic primer sequence forms a primer dimer, a nucleotide residue positioned in a region other than a region to which the primer is complementarily or non-complementarily hybridizable; and (4) when it is predicted that a primer comprising the aforesaid basic primer sequence forms a loop structure in a single primer molecule, a nucleotide residue positioned in a region other than a region which forms the loop structure.

## Description

### Technical Field

The present invention relates to a method for designing a primer having a mutation introduced thereinto.

### Background Art

Currently, nucleic acid amplification methods are vital approaches not only in the field of basic research but in various fields including epidemiologic examination, medical diagnosis, legal medicine and gene analysis. The nucleic acid amplification methods can specifically amplify a target nucleic acid sequence and are therefore very useful as highly sensitive approaches for detecting the presence of the target nucleic acid.

One of the problems associated with the practice of the nucleic acid amplification methods is the formation of a nonspecific amplification product. Furthermore, the formation of a primer dimer or the formation of a loop structure in a single primer molecule is known to be responsible for a nonspecific amplification. The nonspecific amplification caused by the formation of a primer dimer or loop structure may occur, for example, when a 3'-terminal region of a primer has complementarity in itself or with a different primer to some extent.

If such a nonspecific amplification occurs in a nucleic acid amplification system for determining the presence or absence of a target sequence, as in quantitative PCR or an isothermal amplification method, problems such as decrease in S/N ratio and the generation of false positive may arise and preclude the detection of the target sequence.

A method for suppressing the occurrence of a primer dimer, one of the causes of nonspecific amplification reaction, has been proposed under these circumstances.

Patent Literature 1 discloses a method for improving a S/N ratio, comprising using a labeled primer and applying the principles of FRET thereto, thereby preventing a signal caused by a primer dimer from being detected.

Patent Literature 2 discloses a primer comprising, within a 3'-terminal sequence, a modified nucleotide selected from the group consisting of 2'-fluoro-nucleotides, 2'-aminonucleotides and arabinose nucleotides.

As a similar technique, Patent Literature 3 discloses a primer comprising, within a 3'-terminal sequence, an engineered pyrimidine nucleobase.

Patent Literatures 2 and 3 state that use of these primers can suppress the formation of a primer dimer.

### Citation List

### Patent Literature

Patent Literature 1: National Publication of International Patent Application No. 2005-528121
Patent Literature 2: Japanese Patent Laid-Open No. 2002-291490
Patent Literature 3: National Publication of International Patent Application No. 2008-535518

### Summary of Invention

### Technical Problem

The method described in Patent Literature 1 cannot be applied to a system of detecting an amplified nucleic acid using an intercalator, though this approach is effective for quantitative PCR, which involves detecting the presence or absence or abundance of a target nucleic acid by reading out fluorescence emitted from an amplification product. Furthermore, the method cannot suppress the occurrence of a primer dimer itself and therefore, cannot prevent the exhaustion of primers ascribable to the formation of a nonspecific amplification product caused by the primer dimer.

The methods described in Patent Literatures 2 and 3 suppress the occurrence itself of a primer dimer responsible for a nonspecific amplification. These literatures, however, merely abstractly describe "within three 3'-terminal nucleotide positions" or "within 4 nucleotides from the 3' end," etc. as to a position to which a modified or engineered nucleotide is introduced, and do not disclose a method for designing a modified primer which can more effectively suppress a nonspecific amplification.

In light of these circumstances, an object of the present invention is to provide a novel method for designing a mutant primer in which a nonspecific amplification caused by a primer dimer or loop structure scarcely occurs.

### Solution to Problem

To attain the object, the present invention provides a method for designing a primer having a mutation introduced thereinto for use in a nucleic acid amplification method, said method comprising:
a basic sequence-design step for designing, as a basic primer sequence, a nucleotide sequence completely complementary to a template DNA; and
a mutation introduction site-selection step for selecting, as a mutation introduction site, one or more nucleotide residues, said nucleotide residue(s) being contained in the basic primer sequence and satisfying one or more requirements selected from the group consisting of the following requirements (1) to (4):
   (1) a nucleotide residue possibly contributing to the formation of a primer dimer;
   (2) a nucleotide residue possibly contributing to the formation of a loop structure in a single primer molecule;
   (3) when it is predicted that a primer comprising the basic primer sequence forms a primer dimer, a nucleotide residue positioned in a region other than a region to which the primer is complementarily or non-complementarily hybridizable; and
   (4) when it is predicted that a primer comprising the basic primer sequence forms a loop structure in a single primer molecule, a nucleotide residue positioned in a region other than a region which forms the loop structure.

The design method of the present invention can easily design a mutant primer in which a nonspecific amplification caused by a primer dimer or loop structure scarcely occurs.

In the present invention, preferably, the mutation introduced into the mutant primer is not recognized as a nucleotide residue by DNA polymerase.

According to such an embodiment, the design method can design a primer in which a nonspecific amplification more scarcely occurs.

In a preferred embodiment of the present invention, the mutation is one or more mutations selected from the group consisting of the following mutations (A) to (D):
(A) a nucleotide residue or a polynucleotide bound at its 5' end and 3' end with the 5' end and 3' end, respectively, of nucleotide residues flanking the mutation introduction site;
(B) a spacer chain consisting of a carbon chain or a PEG chain;
(C) a spacer chain consisting of a tetrahydrofuran derivative represented by the general formula 1:
   General formula 1 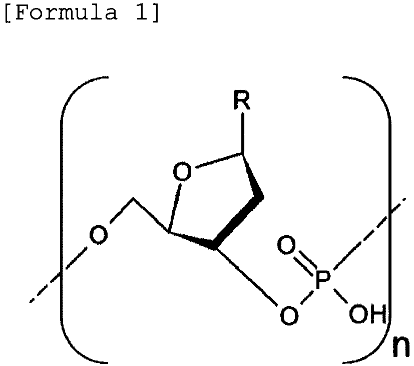 wherein R represents H or a hydroxy group, and n represents a natural number; and
(D) a spacer chain that has undergone photodegradable modification.

The mutations (A) to (D) introduced into the mutant primer permit design of a primer in which a nonspecific amplification much more scarcely occurs.

The present invention also relates to a primer designed by the aforementioned design method and a nucleic acid amplification method using the primer.

The primer and the nucleic acid amplification method of the present invention can reduce a nonspecific amplification in nucleic acid amplification.

The nucleic acid amplification method of the present invention is particularly preferably applied to an isothermal amplification method.

Unlike a PCR method, the isothermal amplification method involves no step of denaturing double-stranded DNA and tends to cause a nonspecific amplification. Therefore, the application of the nucleic acid amplification method of the present invention thereto is particularly preferred.

The present invention also relates to a program for causing a computer to execute each step of the aforementioned design method as a procedure.

The program of the present invention can conveniently design a mutant primer in which a nonspecific amplification scarcely occurs.

### Advantageous Effects of Invention

The present invention can provide a mutant primer and a nucleic acid amplification method in which a nonspecific amplification scarcely occurs.

### Brief Description of Drawings

[Figure 1] Figure 1 is a diagram showing the structure of a normal nucleic acid.
[Figure 2] Figure 2 is a diagram showing the structure of a primer having a mutation (A) introduced thereinto.
[Figure 3] Figure 3 is a diagram showing the structure of a primer having a carbon chain introduced thereinto as a mutation (B).
[Figure 4] Figure 4 is a diagram showing the structure of a primer having a PEG chain introduced thereinto as a mutation (B).
[Figure 5] Figure 5 is a diagram showing the structure of a primer having a spacer chain consisting of a tetrahydrofuran derivative represented by the general formula 1, introduced thereinto as a mutation (C).
[Figure 6] Figure 6 is a diagram showing the structure of a primer having a spacer chain that has undergone photodegradable modification, introduced thereinto as a mutation (D).
[Figure 7] Figure 7 is a schematic diagram of a primer dimer. The filled arrows represent mutation introduction sites. The right and left arrows represent directions in which nucleic acid synthesis is performed. The vertical lines between the two primers represent hydrogen bonds.
[Figure 8] Figure 8 is a schematic diagram of a primer having a mutation introduced thereinto. m represents a mutation. The x marks represent that nucleic acid synthesis is not performed. The vertical line between the two primers represents a hydrogen bond.
[Figure 9] Figure 9 is a schematic diagram of a loop structure. The filled arrows represent mutation introduction sites. The left arrow represents a direction in which nucleic acid synthesis is performed. The vertical lines represent hydrogen bonds.
[Figure 10] Figure 10 is a schematic diagram of a primer having a mutation introduced thereinto. m represents a mutation. The x mark represents that nucleic acid synthesis is not performed. The vertical line between the two primers represents a hydrogen bond.
[Figure 11] Figure 11 is a schematic diagram of a primer dimer. The filled arrows represent mutation introduction sites. The right and left arrows represent directions in which nucleic acid synthesis is performed. The vertical lines between the two primers represent hydrogen bonds.
[Figure 12] Figure 12 is a schematic diagram of a primer having a mutation introduced thereinto. m represents a mutation. The x marks and the right and left arrows represent that nucleic acid synthesis in the direction indicated by the arrow stops at the position indicated by the x mark. The vertical lines between the two primers represent hydrogen bonds.
[Figure 13] Figure 13 is a schematic diagram showing a manner in which a nonspecific amplification product functions as a primer for a new nonspecific amplification resulting in chain reaction of nonspecific amplifications. The filled arrows represent mutation introduction sites. The right and left arrows represent directions in which nucleic acid synthesis is performed. The vertical lines between the two primers represent hydrogen bonds.
[Figure 14] Figure 14 is a schematic diagram showing that the chain reaction of nonspecific amplifications caused by the formation of a primer is suppressed by the introduction of a mutation. m represents a mutation.
[Figure 15] Figure 15 is a schematic diagram of a loop structure. The filled arrow represents a mutation introduction site. The left arrow represents a direction in which nucleic acid synthesis is performed. The vertical lines represent hydrogen bonds.
[Figure 16] Figure 16 is a schematic diagram showing that the chain reaction of nonspecific amplifications caused by the formation of a loop structure is suppressed by the introduction of a mutation. m represents a mutation.
[Figure 17] Figure 17 shows an amplification curve of Test Example 1.
[Figure 18] Figure 18 shows an amplification curve with an expanded time axis of Test Example 1.
[Figure 19] Figure 19 shows an amplification curve of Test Example 2.
[Figure 20] Figure 20 shows an amplification curve with an expanded time axis of Test Example 2.
[Figure 21] Figure 21 is a diagram showing a manner in which a nonspecific amplification occurs by a primer dimer of two F primers to form a nonspecific amplification product P1.
[Figure 22] Figure 22 is a diagram showing a manner in which a nonspecific amplification occurs with the nonspecific amplification product P1 as a primer and an R primer as a template to form a nonspecific amplification product P2.
[Figure 23] Figure 23 is a diagram illustrating a mechanism under which nonspecific amplification reaction is suppressed by primer set 2. This figure shows a manner in which a nonspecific amplification occurs by a primer dimer of two F primers having a mutation introduced thereinto to form a nonspecific amplification product P1'.
[Figure 24] Figure 24 is a diagram illustrating a mechanism under which nonspecific amplification reaction is suppressed by primer set 2. This figure shows a manner in which a nonspecific amplification occurs with the nonspecific amplification product P1' as a primer and an R primer as a template to form a nonspecific amplification product P2'.
[Figure 25] Figure 25 is a diagram illustrating a mechanism under which nonspecific amplification reaction is suppressed by primer set 3. This figure shows a manner in which a nonspecific amplification occurs by a primer dimer of two F primers having a mutation introduced thereinto to form a nonspecific amplification product P1".
[Figure 26] Figure 26 is a diagram illustrating a mechanism under which nonspecific amplification reaction is suppressed by primer set 3. This figure shows a manner in which a nonspecific amplification occurs with the nonspecific amplification product P1" as a primer and an R primer as a template to form a nonspecific amplification product P2".
[Figure 27] Figure 27 shows an amplification curve of Test Example 3.
[Figure 28] Figure 28 shows an amplification curve with an expanded time axis of Test Example 3.
[Figure 29] Figure 29 shows an amplification curve of Test Example 4.
[Figure 30] Figure 30 shows an amplification curve with an expanded time axis of Test Example 4.

### Description of Embodiments

Hereinafter, embodiments of the present invention will be described in detail. However, the technical scope of the present invention is not limited by the embodiments given below.

In the present invention, the nucleic acid amplification method includes all methods for amplifying a nucleic acid and includes: a PCR method; a reverse transcription PCR method, a real-time PCR method, and a DNA sequencing method, which are derived from the PCR method; and isothermal amplification methods such as a LAMP method, a SmartAmp method, and a nucleic acid amplification method described in Re-publication of PCT International Publication No. 2012/124681 (TRIAmp amplification method).

The design method of the present invention is particularly preferably applied to the design of a primer for use in an isothermal amplification method.

In the present invention, the primer refers to a short nucleic acid fragment that plays a role in supplying 3'OH during synthesis of a nucleic acid by DNA polymerase in nucleic acid amplification reaction, and includes DNA and RNA. In the description below, the primer refers to a DNA primer unless otherwise specified.

The present invention provides a method for designing a primer having a mutation introduced thereinto. In the present invention, the phrase "introducing a mutation" means that a nucleotide residue (adenine nucleotide residue, guanine nucleotide residue, thymine nucleotide residue, cytosine nucleotide residue or uracil nucleotide residue) constituting a normal nucleic acid is replaced with a modified nucleotide residue, an engineered nucleotide residue, a chemical structure other than a nucleotide residue, or a nucleotide residue having a binding pattern different from a normal one, etc. The "mutation" refers to any of these chemical structures.

In the present invention, a chemical structure for use as a conventional spacer can be used as the mutation.

Particularly preferred examples of the mutation include chemical structures that are not recognized as a nucleotide residue by DNA polymerase. Examples of such a mutation specifically include the following chemical structures (A) to (D):
(A) a nucleotide residue or a polynucleotide bound at its 5' end and 3' end with the 5' end and 3' end, respectively, of nucleotide residues flanking the mutation introduction site;
(B) a spacer chain consisting of a carbon chain or a PEG chain;
(C) a spacer chain consisting of a tetrahydrofuran derivative represented by the general formula 1; and
(D) a spacer chain that has undergone photodegradable modification.

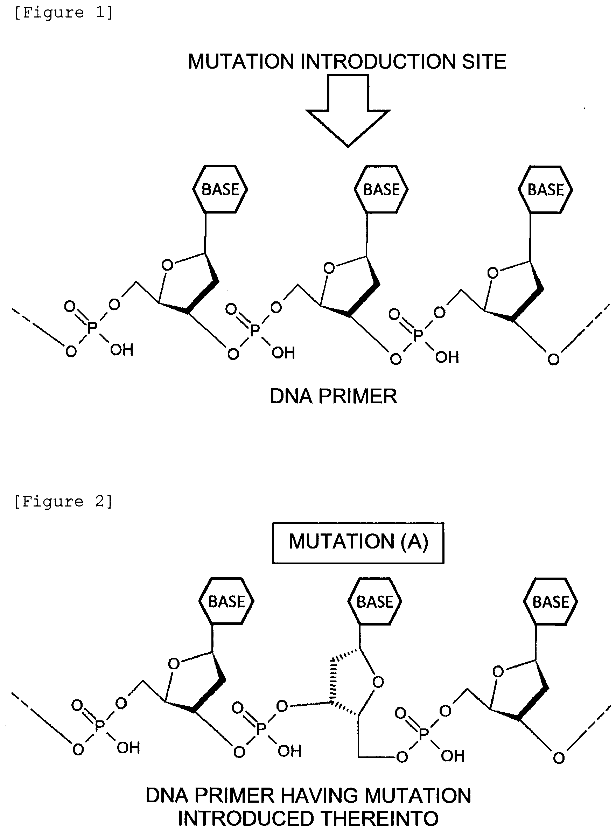

Normally, a nucleotide residue constituting a nucleic acid is bound at its 3' end with the 5' end of a different nucleotide residue and bound at its 5' end with the 3' end of another different nucleotide residue (Figure 1).

The primer having a mutation (A) introduced thereinto is constituted by normal nucleotide residues, whereas the binding pattern of the nucleotide residue or the polynucleotide at the mutation introduction site is reversed with respect to the normal one. Therefore, DNA polymerase cannot recognize this mutation as a nucleotide residue so that nucleic acid synthesis reaction cannot continue (Figure 2). When this primer having a mutation introduced thereinto hybridizes to a complementary strand, the mutation forms no hydrogen bond with a base in the complementary strand.

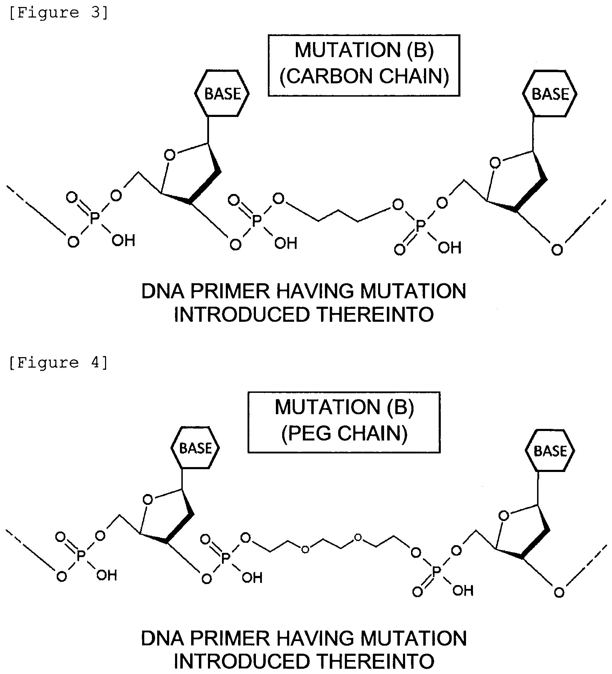

The mutation (B) totally differs in structure from nucleotide residues. Therefore, DNA polymerase cannot recognize this mutation as a nucleotide residue so that nucleic acid synthesis reaction cannot continue (Figures 3 and 4). When this primer having a mutation introduced thereinto hybridizes to a complementary strand, the mutation forms no hydrogen bond with a base in the complementary strand.

In the case of introducing a carbon chain as the mutation, the carbon chain length of the carbon chain per nucleotide residue to be replaced can be preferably 3 to 9.

In the case of introducing a PEG chain as the mutation, the degree of polymerization of the PEG chain per nucleotide residue to be replaced can be preferably 1 to 3.

Examples of the mutation (B) can include a structure derived from 3-(4,4'-dimethoxytrityloxy)propyl-1-[(2-cyanoethyl)-(N,N-diisopropyl)]-phosphoramidite (commonly called Spacer C3), and a structure derived from 8-O-(4,4'-dimethoxytrityl)-triethyleneglycol, 1-[(2-cyanoethyl)-(N,N-diisopropyl)]-phosphoramidite (commonly called Spacer 9).

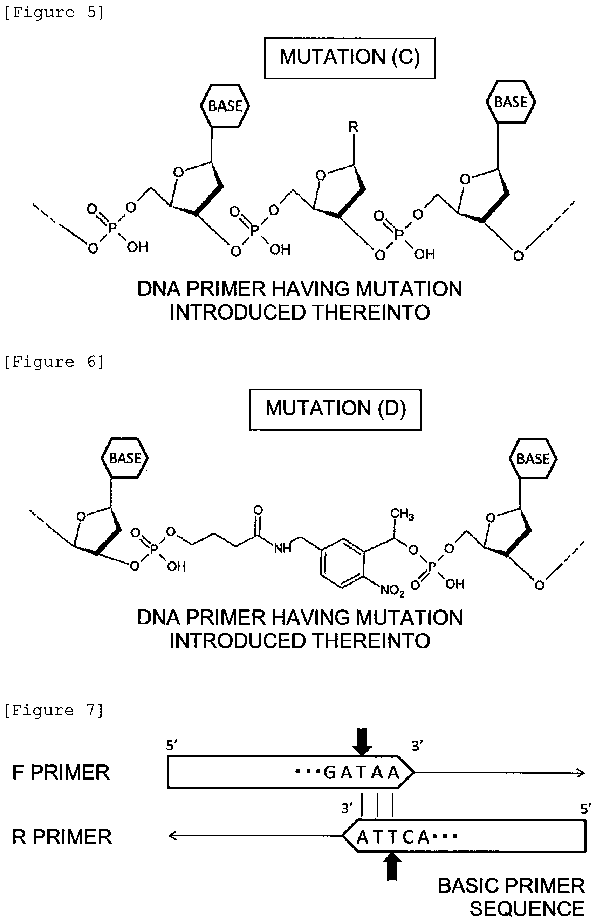

The mutation (C) has no base. Therefore, DNA polymerase cannot recognize this mutation as a nucleotide residue so that nucleic acid synthesis reaction cannot continue (Figure 5). When this primer having a mutation introduced thereinto hybridizes to a complementary strand, the mutation forms no hydrogen bond with a base in the complementary strand.

Examples of the mutation (C) can include a structure derived from 5'-O-dimethoxytrityl-1',2'-dideoxyribose-3'-[(2-cyanoethyl)-(N,N-diisopropyl)]-phosphoramidite (commonly called dSpacer), and a structure derived from 5-O-dimethoxytrityl-1-O-tert-butyldimethylsilyl-2-deoxyribose-3-[(2-cyanoethyl)-(N,N-diisopropyl)]-phosphoramidite (commonly called Abasic).

The TBDMS protective group (tert-butyldimethylsilyl group) in Abasic is deprotected during primer synthesis. Hence, the structure of the primer after the introduction of Abasic is a structure having OH in place of R in Figure 5.

In the case of introducing, as the mutation, a spacer chain consisting of a tetrahydrofuran derivative represented by the general formula 1, the value of n per nucleotide residue to be placed can be preferably 1.

The mutation (D) totally differs in structure from nucleotide residues. Therefore, DNA polymerase cannot recognize this mutation as a nucleotide residue so that nucleic acid synthesis reaction cannot continue (Figure 6). When this primer having a mutation introduced thereinto hybridizes to a complementary strand, the mutation forms no hydrogen bond with a base in the complementary strand.

The spacer chain that has undergone photodegradable modification is a spacer chain modified with a compound having the property of being degraded by exposure to UV or visible light, and refers to a compound group, a feature of which is to have a nitrobenzene skeleton in the present specification (see Japanese Patent No. 4870791).

Examples of the mutation (D) can include a structure derived from 1-[2-nitro-5-(6-trifluoroacetylcaproamidomethyl)phenyl]-ethyl-[2-cyanoethyl(N,N-diisopropyl)]phosphoramidite (commonly called PC 5'-Amino-Modifier), a structure derived from 1-[2-nitro-5-(6-(N-(4,4'-dimethoxytrityl))biotinamidocaproamidomethyl)phenyl]-ethyl-[2-cyanoethyl-(N,N-diisopropyl)]phosphoramidite (commonly called PC 5'-Biotin), a structure derived from 3-(4,4'-dimethoxytrityl)-1-(2-nitrophenyl)-propane-1,3-diol-[2-cyanoethyl-(N,N-diisopropyl)]phosphoramidite (commonly called PC Linker), and a structure derived from [4-(4,4'-dimethoxytrityloxy)butyramidomethyl)-1-(2-nitrophenyl)-ethyl]-2-cyanoethyl-(N,N-diisopropyl)-phosphoramidite (commonly called PC Spacer).

The number of mutations to be introduced into one primer can be appropriately set without losing the specificity of the primer. The specificity of the primer can be calculated using a general primer design tool.

Next, each step of the design method of the present invention will be described.

The method for designing a primer according to the present invention comprises a basic sequence-design step. The basic sequence-design step is the step for designing, as a basic primer sequence, a nucleotide sequence completely complementary to a template DNA, and can be performed in the same way as in primer design in an ordinary nucleic acid amplification method. Specifically, a nucleotide sequence of approximately 15 to 50 bases suitable for the primer is selected from within a target nucleotide sequence from the viewpoint of specificity, a GC content, a Tm value, etc. Methods for calculating specificity, a GC content, a Tm value, etc. are not limited and may be manual calculation or may employ a calculation tool or the like generally used.

The number of basic primer sequences to be designed for one system is appropriately changed depending on the system of the nucleic acid amplification method of interest. Specifically, when the nucleic acid amplification method of interest is an ordinary PCR method, two primers (F primer and R primer) are designed. When the nucleic acid amplification method of interest is a system that requires three or more primers, as in a Lamp method, three or more basic primer sequences are designed.

A mutation introduction site-selection step is performed after the design of the basic primer sequence in the basic sequence-design step. The mutation introduction site-selection step is the step for selecting nucleotide residue(s) from the basic primer sequence and using the nucleotide residue(s) as a mutation introduction site, the nucleotide residue(s) satisfying one or more requirements selected from the group consisting of the following requirements (1) to (4) :
(1) a nucleotide residue possibly contributing to the formation of a primer dimer;
(2) a nucleotide residue possibly contributing to the formation of a loop structure in a single primer molecule;
(3) when it is predicted that a primer comprising the basic primer sequence forms a primer dimer, a nucleotide residue positioned in a region other than a region to which the primer is complementarily or non-complementarily hybridizable; and
(4) when it is predicted that a primer comprising the basic primer sequence forms a loop structure in a single primer molecule, a nucleotide residue positioned in a region other than a region which forms the loop structure.

An approach for predicting whether or not the primer comprising the basic primer sequence forms a primer dimer or loop structure, or predicting which nucleotide residues contribute to the formation is not particularly limited and may employ a calculation tool or the like generally used.

First, a nucleotide residue satisfying the requirement (1) is a nucleotide residue possibly contributing to the formation of a primer dimer, i.e., possibly forming a hydrogen bond with a base in a complementary strand, and is, for example, a nucleotide residue shown in Figure 7 (TAA of an F primer or TTA of an R primer). Upon formation of a primer dimer, the primer itself serves as a template resulting in a nonspecific amplification, as shown in Figure 7.

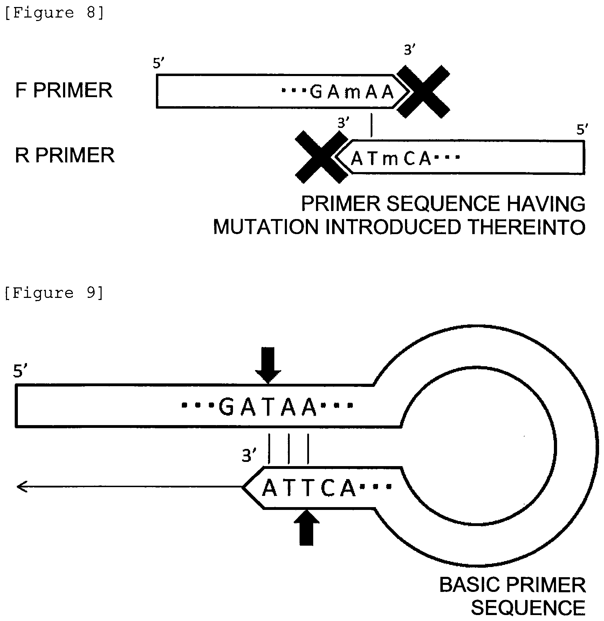

The nucleotide residue satisfying the requirement (1) is selected as a mutation introduction site (indicated by filled arrow in Figure 7), and a mutation is introduced into this site. As a result, the formation of a primer dimer can be suppressed, and the occurrence of a nonspecific amplification can be suppressed (Figure 8) .

Figures 7 and 8 summarize the suppression of the formation of a primer dimer between primers different from each other, i.e., an F primer and an R primer. As a matter of course, a nucleotide residue contributing to the formation of a primer dimer between the same primers also satisfies the requirement (1).

A nucleotide residue satisfying the requirement (2) is a nucleotide residue possibly contributing to the formation of a loop structure, and is, for example, a nucleotide residue shown in Figure 9 (TAA on the 5' side or TTA on the 3' side). Upon formation of a loop structure, the primer itself serves as a template resulting in a nonspecific amplification, as shown in Figure 9.

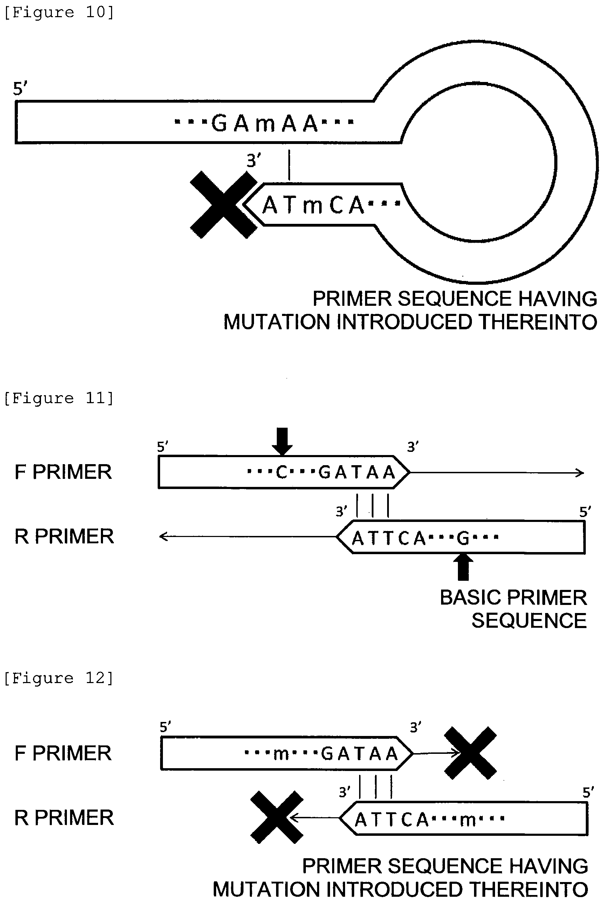

The nucleotide residue satisfying the requirement (2) is selected as a mutation introduction site (indicated by filled arrow in Figure 9), and a mutation is introduced into this site. As a result, the formation of a loop structure can be suppressed, and the occurrence of a nonspecific amplification can be suppressed (Figure 10) .

A nucleotide residue satisfying the requirement (3) is, when it is predicted that a primer comprising the basic primer sequence forms a primer dimer, a nucleotide residue positioned in a region other than a region to which the primer is complementarily or non-complementarily hybridizable, and is, for example, a nucleotide residue shown in Figure 11 (C of an F primer or G of an R primer).

Upon formation of a primer dimer, the primer itself serves as a template resulting in a nonspecific amplification up to the maximum strand length synthesizable by DNA polymerase, as shown in Figure 11. The nucleotide residue satisfying the requirement (3) is selected as a mutation introduction site (indicated by filled arrow in Figure 11), and a mutation is introduced into this site. As a result, DNA synthesis reaction mediated by DNA polymerase can be inhibited at the mutation introduction site, and a nonspecific amplification product can be controlled to a short strand length (Figure 12).

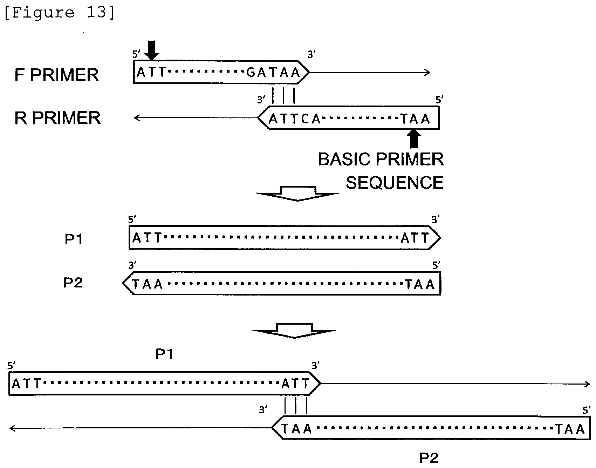

The selection of a nucleotide residue satisfying the requirement (3) as a mutation introduction site is particularly effective, for example, when a nonspecific amplification product is capable of functioning as a primer for a new nonspecific amplification, as shown in Figure 13. Nonspecific amplification products P1 and P2 formed by the primers shown in Figure 13 are denatured into single strands, followed by re-hybridization between the single strands to cause a new nonspecific amplification. A product of this new nonspecific amplification is capable of functioning as a primer that causes a further new nonspecific amplification. Therefore, chain reaction of nonspecific amplifications does not stop.

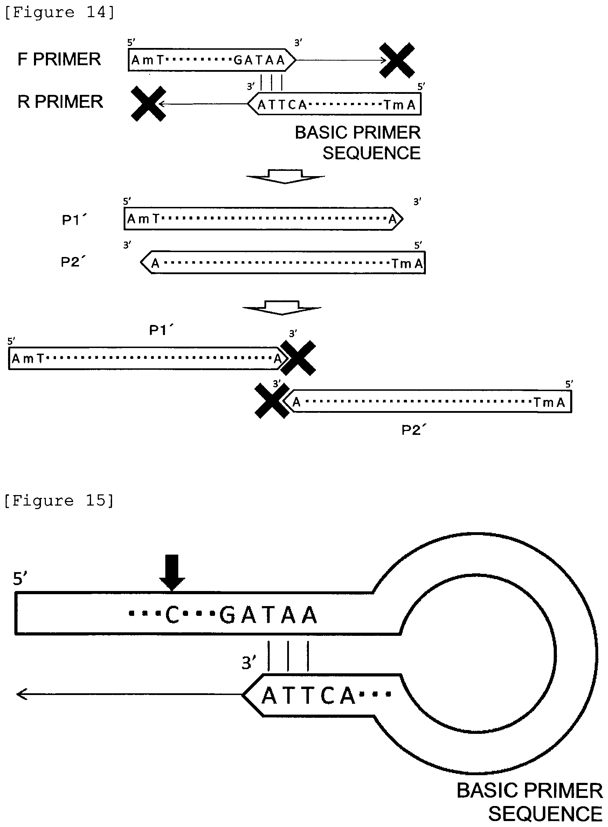

In this case, a mutation is introduced into the nucleotide residue satisfying the requirement (3). As a result, chain reaction of nonspecific amplifications can be prevented, as shown in Figure 14, because nonspecific amplification products P1' and P2' do not function as a primer for a new nonspecific amplification.

Figures 13 and 14 summarize the suppression of the formation of a primer dimer between primers different from each other, i.e., an F primer and an R primer. As a matter of course, a nucleotide residue contributing to the formation of a primer between the same primers also satisfies the requirement (3).

A nucleotide residue satisfying the requirement (4) is, when it is predicted that a primer comprising the basic primer sequence forms a loop structure in a single primer molecule, a nucleotide residue positioned in a region other than a region which forms the loop structure, and is, for example, a nucleotide residue (C) shown in Figure 15. In this context, the "region which forms the loop structure" refers to a loop-shaped sequence region, and a region to which the primer is complementarily or non-complementarily hybridizable.

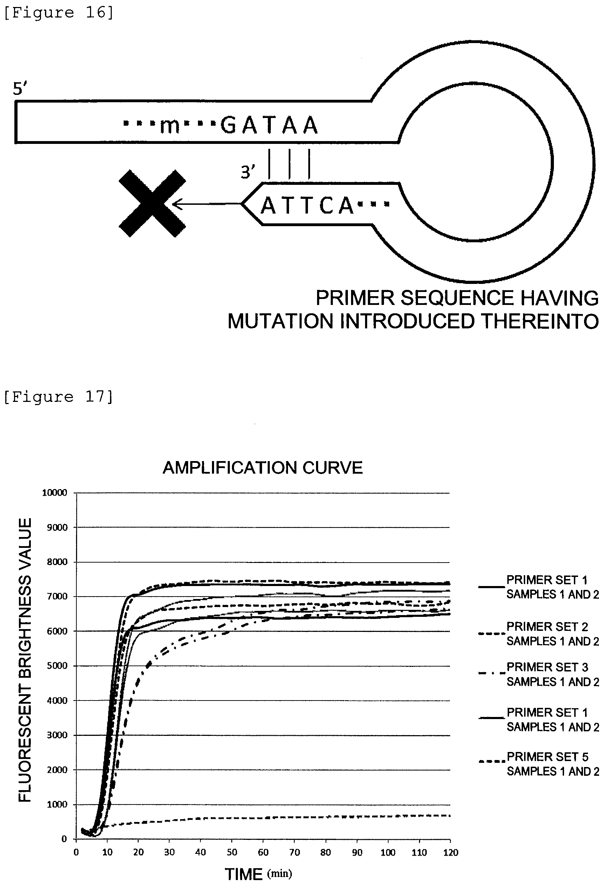

Upon formation of a loop structure, the primer itself serves as a template resulting in a nonspecific amplification up to the maximum strand length synthesizable by DNA polymerase, as shown in Figure 15. The nucleotide residue satisfying the requirement (4) is selected as a mutation introduction site (indicated by filled arrow in Figure 15), and a mutation is introduced into this site. As a result, DNA synthesis reaction mediated by DNA polymerase can be inhibited at the mutation introduction site, and a nonspecific amplification product can be controlled to a short strand length (Figure 16).

The selection of a nucleotide residue satisfying the requirement (4) as a mutation introduction site is particularly effective when a nonspecific amplification product is capable of functioning as a primer for a new nonspecific amplification, under the same principles as those described with reference to Figures 13 and 14. Chain reaction of nonspecific amplifications can be prevented by introducing a mutation into the nucleotide residue satisfying the requirement (4).

The primer designed by the design method of the present invention may be variously modified for a purpose other than the suppression of a nonspecific amplification.

### Examples

### <Test Example 1>

In order to amplify a direct repeat sequence in the genomic DNA of a Mycobacterium bovis BCG str. Tokyo 172 strain by the nucleic acid amplification method described in Re-publication of PCT International Publication No. 2012/124681 (TRIAmp amplification method), a set of primers designated as DRa-21 and DRb-19 in the literature was provided. The sequences of these primers are shown as primer 1F and primer 1R in the upper rows of Table 1. A solvent for the primers described in Test Examples of the present invention is a 1× TE buffer unless otherwise specified.

Nucleotide residues possibly contributing to the formation of a hairpin loop, a homodimer or a heterodimer in the sequences of the primers 1F and 1R were selected using a general calculation program available on the web or available by download.

The nucleotide residues thus selected were selected as mutation introduction sites, and primers of primer sets 2 and 3 shown in Table 1 were designed and prepared.

Primers consisting of a sequence 3' to the mutation introduction site in primer sets 2 and 3 were also provided (primer sets 4 and 5, Table 1).

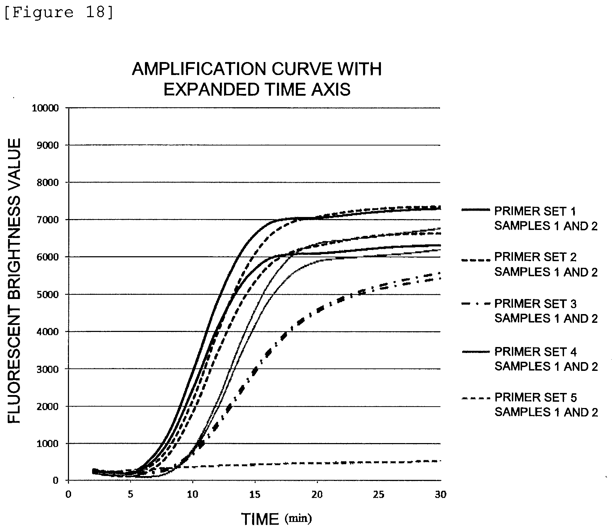

The reaction solution shown in Table 2 was prepared using each primer set of F and R primers shown in Table 1, and the TRIAmp amplification method was performed. The TRIAmp amplification reaction was performed at 68°C for 2 hours using Thermal Cycler Dice Real Time System Lite TP710, and the reaction was monitored on a FAM detection mode. The consequently calculated amplification curve and Ct value are shown in Figures 17 and 18 and Table 3, respectively. For the TRIAmp amplification reaction, two sets of the reaction solution were prepared for each primer set (hereinafter, these samples are referred to as samples 1 and 2), and the amplification curve and the Ct value were calculated for each of these sets.

### [Table 1]

**[Table 1]**

| Primer set | Primer name | Sequence | SEQ ID NO |
|---|---|---|---|
| 1 | Primer 1F | CGGGGTTTTGGGTCTGACGAC | 1 |
| | Primer 1R | CCCGAGAGGGGACGGAAAC | 2 |
| 2 | Primer 2F | CG*GGGTTTTGGGTGTGACGAC | - |
| | Primer 2R | CC*CGAGAGGGGACGGAAAO | - |
| 3 | Primer 3F | CGGGGTTTTGGG*TCTGACGAC | - |
| | Primer 3R | CCCGAGAGGGG*ACGGAAAC | - |
| 4 | Primer 4F | GGGTTTTGGGTCTGACGAC | 3 |
| | Primer 4R | CGAGAGGGGACGGAAAC | 4 |
| 5 | Primer 5F | TCTGACGAC | - |
| | Primer 5R | ACGGAAAC | - |

| | | | |
|---|---|---|---|
| Primer set 1: unmodified primers Primer sets 2 and 3: primers having a mutation introduced thereinto. A nucleotide residue with upper right * represents a nucleotide residue bound at its 3' end and 5' end with the 3' end and 5' end, respectively, of flanking nucleotide residues. Primer set 4: primers consisting of a sequence 3' to the mutation introduction site of primer set 2. Primer set 5: primers consisting of a sequence 3' to the mutation introduction site of primer set 3. | | | |

### [Table 2]

**[Table 2]**

| Component | Final concentration |
|---|---|
| THs-HCl (pH8.8) | 20 mM |
| KCl | 10 mM |
| MgSO₄ | 7.2 mM |
| F Primer | 2 *µ* M |
| R Primer | 2 *µ* M |
| (NH₄)₂SO₄ | 30 mM |
| BSA | 3.2 *µ* g/ *µ* L |
| dNTPmix | 1.4 mM |
| Bst Polymerase 3.0 | 0.32 U/ *µ* L |
| Fluorescent reagent *1 | 0.04 *µ* L/ *µ* L |
| Template DNA *2 | 0.2 pg/ *µ* L |
| Water | up to 25 *µ* L |

| | |
|---|---|
| *1: prepared by adding 3.5 µL of GelGreen(TM) (10,000× DMSO solution, Biotium, Inc.) to 1 mL of a 3 mM HNB (2-hydroxy-1-(2-hydroxy-4-sulfo-1-naphthylazo)-3,6-naphthalenedisulfonic acid, trisodium salt) solution. *2: Template DNA: genomic DNA of a *Mycobacterium bovis* BCG str. Tokyo 172 strain | |

### [Table 3]

**[Table 3]**

| Primer set | Sample name | Ct value (min) |
|---|---|---|
| 1 | Sample 1 | 7.2 |
| | Sample 2 | 7.0 |
| 2 | Sample 1 | 7.4 |
| | Sample 2 | 7.2 |
| 3 | Sample 1 | 8.9 |
| | Sample 2 | 9.0 |
| 4 | Sample 1 | 9.5 |
| | Sample 2 | 9.3 |
| 5 | Sample 1 | - |
| | Sample 2 | - |

As shown in Figures 17 and 18 and Table 3, all the primers used except for primer set 5 having an exceedingly short primer length had an equivalent amplification rate, though somewhat differing in final brightness.

These results indicate that a primer having a mutation introduced thereinto by the method of the present invention differs in function from a primer merely lacking the mutation introduction site and a region 5' thereto in this primer.

### <Test Example 2>

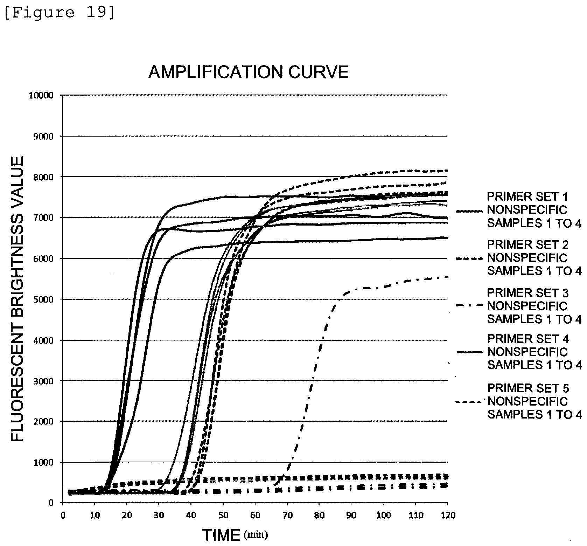

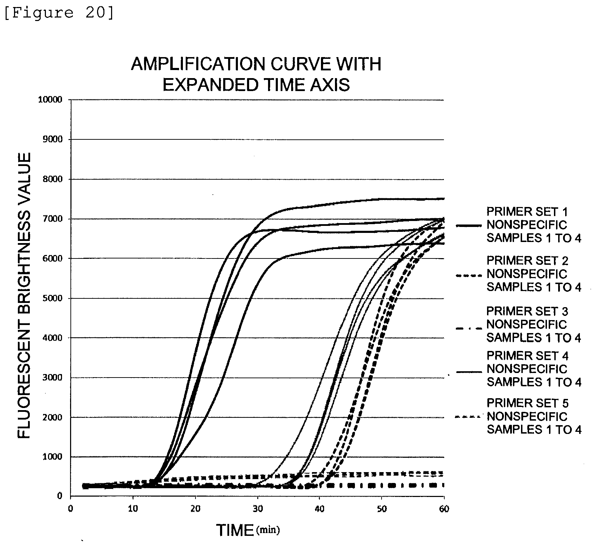

A reaction solution containing water in place of a template DNA was prepared according to the composition shown in Table 2 using each primer set of F and R primers shown in Table 1. TRIAmp amplification reaction was performed under the same conditions as in Test Example 1 to calculate an amplification curve and a Ct value. The results are shown in Table 4 and Figures 19 and 20.

For the TRIAmp amplification reaction, four sets of the reaction solution were prepared for each primer set (hereinafter, these samples are referred to as nonspecific samples 1 to 4), and the amplification curve and the Ct value were calculated for each of these sets.

### [Table 4]

**[Table 4]**

| Primer set | Sample name | Ct value (min) |
|---|---|---|
| 1 | Nonspecific sample 1 | 15.4 |
| | Nonspecific sample 2 | 17.4 |
| | Nonspecific sample 3 | 16.0 |
| | Nonspecific sample 4 | 16.4 |
| 2 | Nonspecific sample 1 | 43.1 |
| | Nonspecific sample 2 | 42.4 |
| | Nonspecific sample 3 | 41.1 |
| | Nonspecific sample 4 | 43.3 |
| 3 | Nonspecific sample 1 | 67.7 |
| | Nonspecific sample 2 | - |
| | Nonspecific sample 3 | - |
| | Nonspecific sample 4 | - |
| 4 | Nonspecific sample 1 | 37.9 |
| | Nonspecific sample 2 | 37.8 |
| | Nonspecific sample 3 | 34.3 |
| | Nonspecific sample 4 | 38.5 |
| 5 | Nonspecific sample 1 | - |
| | Nonspecific sample 2 | - |
| | Nonspecific sample 3 | - |
| | Nonspecific sample 4 | - |

As shown in Figures 19 and 20 and Table 4, a nucleic acid amplification rate in a system free from a template DNA, i.e., a nonspecific amplification rate, is found to be much slower in the case of using primer sets 2 and 3 having a mutation introduced thereinto as compared with the case of using primer set 1 having no mutation introduced thereinto. Particularly, in the case of using primer set 3, the nonspecific nucleic acid amplification did not occur in 3 out of the four samples.

These results indicate that a nonspecific amplification is strongly suppressed by the introduction of a mutation into a primer.

Primer set 4 has a sequence 3' to the mutation introduction site in primer set 2. As shown in Table 4, a nonspecific amplification rate is slower in the case of performing TRIAmp amplification reaction using primer set 4 as compared with the case of using primer set 1. However, an effect of suppressing the nonspecific amplification rate is more strongly exhibited by use of primer set 2 than by use of primer set 4 (Table 4).

Primer set 5 has a sequence 3' to the mutation introduction site in primer set 3. In the case of using primer 5, nucleic acid amplification reaction does not occur even if the reaction solution contained a template DNA.

These results indicate that a primer having a mutation introduced thereinto by the method of the present invention differs in function from a primer merely lacking the mutation introduction site and a region 5' thereto in this primer.

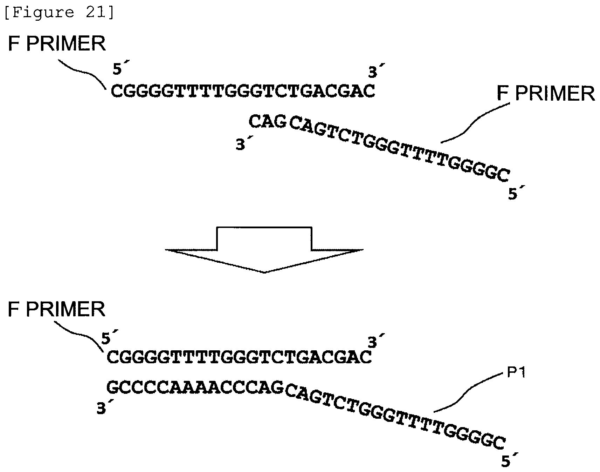

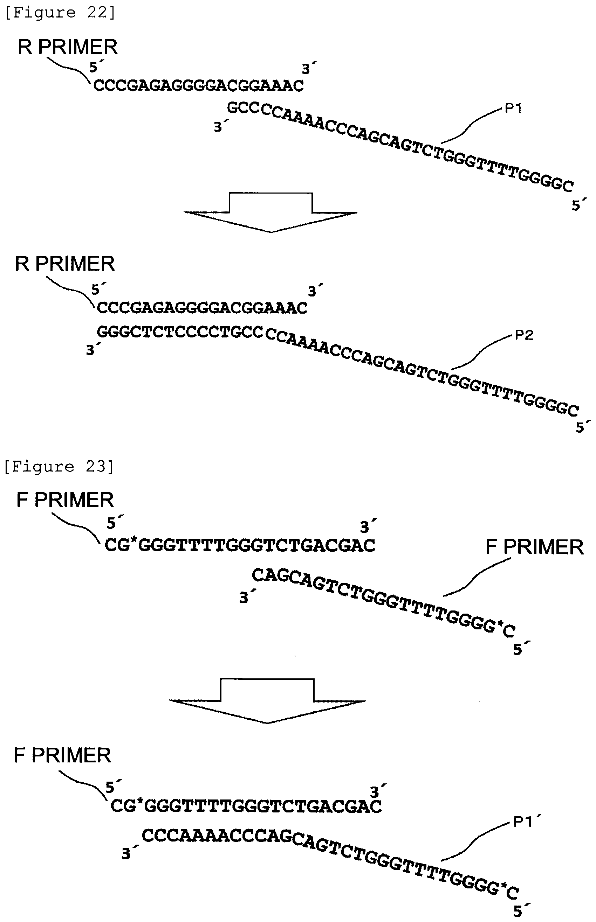

A nonspecific amplification in TRIAmp amplification reaction using primer set 1 is presumably caused by the mechanisms shown in Figures 21 and 22. Specifically, an F primer forms a primer dimer with another F primer (upper area of Figure 21). As DNA synthesis reaction proceeds, a nonspecific amplification product P1 appears (lower area of Figure 21). Subsequently, a single strand of the denatured nonspecific amplification product P1 forms a dimer with an R primer (upper area of Figure 22). As DNA synthesis reaction proceeds, a nonspecific amplification product P2 appears (lower area of Figure 22). Then, nonspecific amplifications proceed one after another because a sequence (CCC) complementary to three nucleotides (GGG) at the 3' end of P2 is present in P1 or P2.

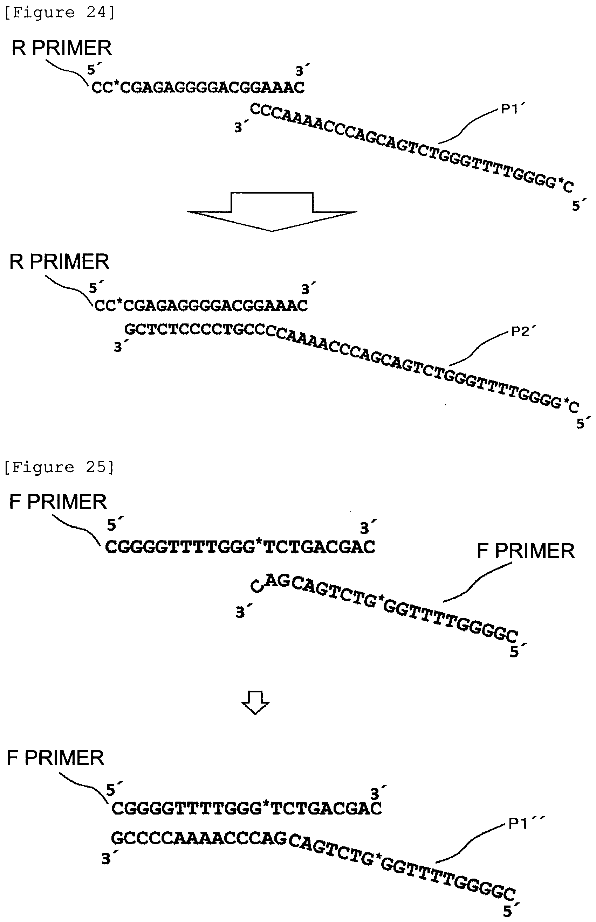

On the other hand, in the case of using primer set 2, even if DNA synthesis reaction proceeds through the formation of a primer dimer between an F primer and another F primer (upper area of Figure 23), its elongation stops at the third nucleotide residue (G) counted from the 5' end of the templated F primer (lower area of Figure 23). Therefore, P1' resulting from this nonspecific amplification has the difficulty in forming, at its 3' end, a dimer with an R primer so that chain reaction of nonspecific amplifications does not occur. Even if DNA synthesis reaction occurs through the formation of a dimer between P1' and an R primer (upper area of Figure 24), its elongation stops at the third nucleotide residue (C) counted from the 5' end of the templated R primer (lower area of Figure 24). Therefore, P2' resulting from this nonspecific amplification is unlikely to further cause chain reaction of nonspecific amplifications.

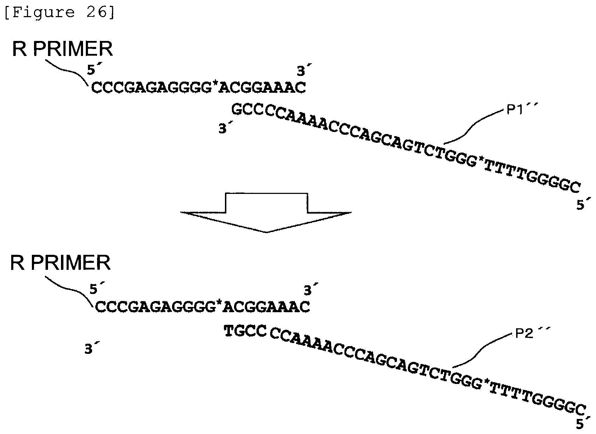

In the F primer of primer set 3, a mutation was introduced into a nucleotide residue contributing to the formation of a primer dimer by two F primers. Thus, a primer is scarcely formed, and DNA synthesis reaction very scarcely occurs (upper area of Figure 25). Even if DNA synthesis reaction occurs to form an amplification product P1" (lower area of Figure 25), which in turn forms a primer dimer with an R primer to cause DNA synthesis reaction (upper area of Figure 26), this reaction stops at the mutation introduction site in the R primer (lower area of Figure 26). Therefore, the amplification product P2" is unlikely to further cause chain reaction of nonspecific amplifications.

For these reasons, primer sets 2 and 3 consisting of primers having a mutation introduced thereinto are considered to have an effect of suppressing a nonspecific amplification reaction rate.

### <Test Example 3>

Primer sets of F and R primers in which a structure derived from Spacer C3, dSpacer, Spacer 9, Abasic or PC Spacer was introduced to a position represented by m in the sequences shown in Table 5 were provided.

### [Table 5]

**[Table 5]**

| Primer set | Name | Sequence | Modification method |
|---|---|---|---|
| 3 Spacer C3 | Primer 3 Spacer C3 F | CGGGGTTTTGGmTCTGACGAC | Spacer C3 |
| | Primer 3 Spacer C3 R | CCCGAGAGGGmACGGAAAC | |
| 3 dSpacer | Primer 3 dSpacer F | CGGGGTTTTGGmTCTGACGAC | dSpacer |
| | Primer 3 dSpacer R | CCCGAGAGGGmACGGAAAC | |
| 3 Spacer 9 | Primer 3 Spacer 9 F | CGGGGTTTTGGmTCTGACGAC | Spacer 9 |
| | Primer 3 Spacer 9 R | CCCGAGAGGGmACGGAAAC | |
| 3 Abasic | Primer 3 Abasic F | CGGGGTTTTGGmTCTGACGAC | Abasic |
| | Primer 3 Abasic R | CCCGAGAGGGmACGGAAAC | |
| 3 PC Spacer | Primer 3 PC Spacer F | CGGGGTTTTGGmTCTGACGAC | PC Spacer |
| | Primer 3 PC Spacer R | CCCGAGAGGGmACGGAAAC | |

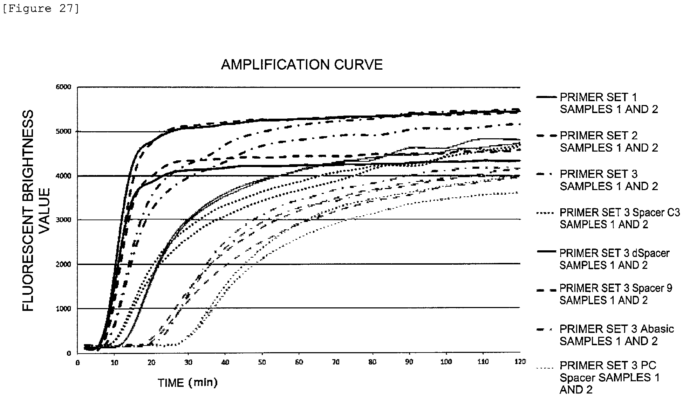

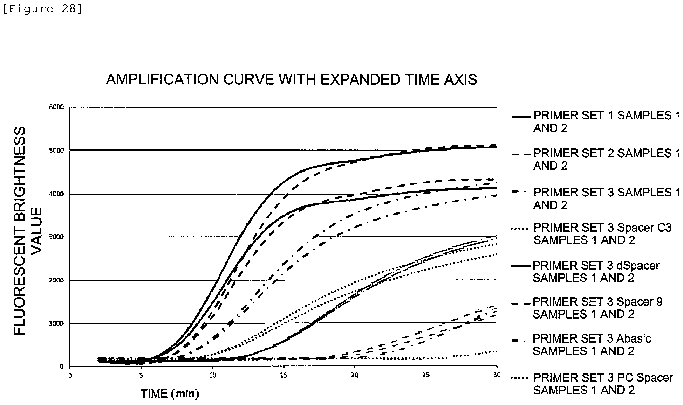

TRIAmp amplification reaction was performed under the same conditions as in Test Example 1 according to the composition shown in Table 2 using the primer sets of F and R primers shown in Table 5 and primer sets 1 to 3 of F and R primers shown in Table 1 to calculate an amplification curve and a Ct value. The results are shown in Table 6 and Figures 27 and 28.

A solvent for primer set 3 Abasic was a 0.2 M solution of triethylamine in acetic acid diluted into 10 µM with sterilized water.

### [Table 6]

**[Table 6]**

| Primer set | Sample name | Ct value (min) |
|---|---|---|
| 1 | Primer set 1 Sample 1 | 6.2 |
| | Primer set 1 Sample 2 | 6.1 |
| 2 | Primer set 2 Sample 1 | 6.7 |
| | Primer set 2 Sample 2 | 6.6 |
| 3 | Primer set 3 Sample 1 | 8.5 |
| | Primer set 3 Sample 2 | 8.3 |
| 3 Spacer C3 | Primer set 3 Spacer C3 Sample 1 | 10.1 |
| | Primer set 3 Spacer C3 Sample 2 | 10.3 |
| 3 dSpacer | Primer set 3 dSpacer Sample 1 | 13.4 |
| | Primer set 3 dSpacer Sample 2 | 13.1 |
| 3 Spacer 9 | Primer set 3 Spacer 9 Sample 1 | 20.1 |
| | Primer set 3 Spacer 9 Sample 2 | 19.7 |
| 3 Abasic | Primer set 3 Abasic Sample 1 | 22.9 |
| | Primer set 3 Abasic Sample 2 | 23.1 |
| 3 PC Spacer | Primer set 3 PC Spacer Sample 1 | 29.2 |
| | Primer set 3 PC Spacer Sample 2 | 30.1 |

As shown in Table 6 and Figures 27 and 28, DNA amplification was able to be performed without problems using any primer set having a Spacer C3-, dSpacer-, Spacer 9-, Abasic- or PC Spacer-derived mutation introduced thereinto, though its amplification rate was somewhat slower than that for primer sets 1 to 3.

### <Test Example 4>

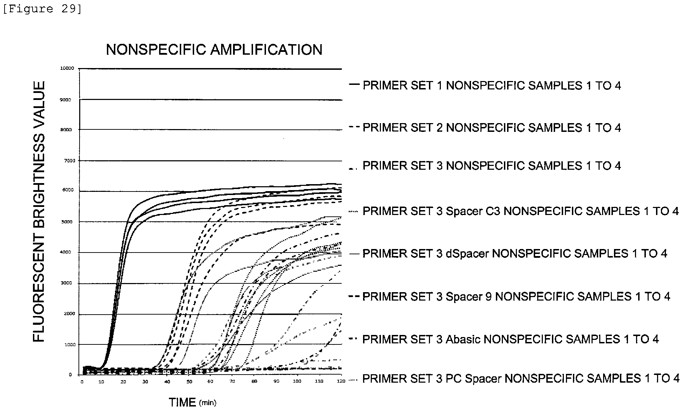

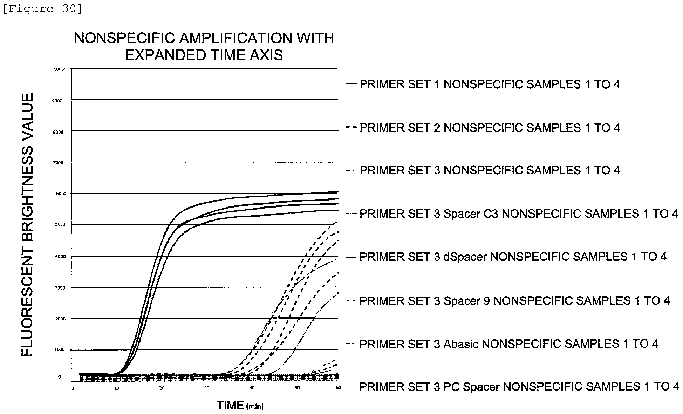

A reaction solution containing water in place of a template DNA was prepared according to the composition shown in Table 2 using the primer sets of F and R primers shown in Table 5 and primer sets 1 to 3 of F and R primers shown in Table 1. TRIAmp amplification reaction was performed under the same conditions as in Test Example 1 to calculate an amplification curve and a Ct value. The results are shown in Table 7 and Figures 29 and 30.

For the TRIAmp amplification reaction, four sets of the reaction solution were prepared for each primer set (hereinafter, these samples are referred to as nonspecific samples 1 to 4), and the amplification curve and the Ct value were calculated for each of these sets.

### [Table 7]

**[Table 7]**

| Primer set | Sample name | Ct value (min) |
|---|---|---|
| 1 | Primer set 1 nonspecific sample 1 | 11.7 |
| | Primer set 1 nonspecific sample 2 | 12.0 |
| | Primer set 1 nonspecific sample 3 | 11.4 |
| | Primer set 1 nonspecific sample 4 | 10.9 |
| 2 | Primer set 2 nonspecific sample 1 | 38.8 |
| | Primer set 2 nonspecific sample 2 | 38.1 |
| | Primer set 2 nonspecific sample 3 | 37.0 |
| | Primer set 2 nonspecific sample 4 | 42.1 |
| 3 | Primer set 3 nonspecific sample 1 | |
| | Primer set 3 nonspecific sample 2 | 62.7 |
| | Primer set 3 nonspecific sample 3 | 97.5 |
| | Primer set 3 nonspecific sample 4 | 56.4 |
| 3 Spacer C3 | Primer set 3 Spacer C3 nonspecific sample 1 | 75.4 |
| | Primer set 3 Spacer C3 nonspecific sample 2 | 62.5 |
| | Primer set 3 Spacer C3 nonspecific sample 3 | 67.3 |
| | Primer set 3 Spacer C3 nonspecific sample 4 | 65.5 |
| 3 dSpacer | Primer set 3 dSpacer nonspecific sample 1 | 36.5 |
| | Primer set 3 dSpacer nonspecific sample 2 | 45.9 |
| | Primer set 3 dSpacer nonspecific sample 3 | 57.8 |
| | Primer set 3 dSpacer nonspecific sample 4 | 61.8 |
| 3 Spacer 9 | Primer set 3 Spacer 9 nonspecific sample 1 | - |
| | Primer set 3 Spacer 9 nonspecific sample 2 | - |
| | Primer set 3 Spacer 9 nonspecific sample 3 | - |
| | Primer set 3 Spacer 9 nonspecific sample 4 | 101.7 |
| 3 Abasic | Primer set 3 Abasic nonspecific sample 1 | 75.6 |
| | Primer set 3 Abasic nonspecific sample 2 | 54.2 |
| | Primer set 3 Abasic nonspecific sample 3 | - |
| | Primer set 3 Abasic nonspecific sample 4 | 77.3 |
| 3 PC Spacer | Primer set 3 PC Spacer nonspecific sample 1 | - |
| | Primer set 3 PC Spacer nonspecific sample 2 | - |
| | Primer set 3 PC Spacer nonspecific sample 3 | - |
| | Primer set 3 PC Spacer nonspecific sample 4 | - |

As shown in Figures 27 to 30 and Tables 6 and 7, a nucleic acid amplification rate in a system free from a template DNA, i.e., a nonspecific amplification rate, is also found to be much slower in the case of using primer sets in which a spacer chain consisting of a carbon chain or a PEG chain, a spacer chain consisting of a tetrahydrofuran derivative represented by the general formula 1, or a spacer chain that underwent photodegradable modification was introduced as a mutation, as compared with primer set 1 having no mutation introduced therein.

These results indicate that a nonspecific amplification is strongly suppressed by the introduction of a mutation into a primer by the method of the present invention.

### Industrial Applicability

The present invention can be applied to a method for reducing a nonspecific amplification in a nucleic acid amplification method.

## Claims

1. A method for designing a primer having a mutation introduced thereinto for use in a nucleic acid amplification method, said method comprising:
a basic sequence-design step for designing, as a basic primer sequence, a nucleotide sequence completely complementary to a template DNA; and
a mutation introduction site-selection step for selecting, as a mutation introduction site, one or more nucleotide residues, said nucleotide residue(s) being contained in the basic primer sequence and satisfying one or more requirements selected from the group consisting of the following requirements (1) to (4):
(1) a nucleotide residue possibly contributing to the formation of a primer dimer;
(2) a nucleotide residue possibly contributing to the formation of a loop structure in a single primer molecule;
(3) when it is predicted that a primer comprising the aforesaid basic primer sequence forms a primer dimer, a nucleotide residue positioned in a region other than a region to which the primer is complementarily or non-complementarily hybridizable; and
(4) when it is predicted that a primer comprising the aforesaid basic primer sequence forms a loop structure in a single primer molecule, a nucleotide residue positioned in a region other than a region which forms the loop structure.

2. The design method according to claim 1, wherein the aforesaid mutation is not recognized as a nucleotide residue by DNA polymerase.

3. The design method according to claim 1 or 2, wherein the aforesaid mutation is one or more mutations selected from the group consisting of the following mutations (A) to (D):
(A) a nucleotide residue or a polynucleotide bound at its 5' end and 3' end with the 5' end and 3' end, respectively, of nucleotide residues flanking the aforesaid mutation introduction site;
(B) a spacer chain consisting of a carbon chain or a PEG chain;
(C) a spacer chain consisting of a tetrahydrofuran derivative represented by the general formula 1:
General formula 1 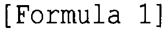 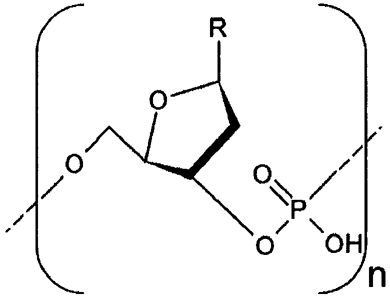 wherein R represents H or a hydroxy group, and n represents a natural number; and
(D) a spacer chain that has undergone photodegradable modification.

4. A primer designed by a design method according to any one of claims 1 to 3.

5. A nucleic acid amplification method comprising using a primer according to claim 4.

6. The nucleic acid amplification method according to claim 5, wherein the nucleic acid amplification method is an isothermal amplification method.

7. A program for causing a computer to execute each step of a design method according to any one of claims 1 to 3 as a procedure.


SEQUENCE LISTING

<110>  Daikeniki Kabushiki Kaisha

<120>  The Design Method of the Mutated Primer.

<130>  P00661

<160>  4

<170>  PatentIn version 3.5

<210>  1
<211>  21
<212>  DNA
<213>  Mycobacterium bovis

<400>  1
cggggttttg ggtctgacga c                                                21


<210>  2
<211>  19
<212>  DNA
<213>  Mycobacterium bovis

<400>  2
cccgagaggg gacggaaac                                                   19


<210>  3
<211>  19
<212>  DNA
<213>  Mycobacterium bovis

<400>  3
gggttttggg tctgacgac                                                   19


<210>  4
<211>  17
<212>  DNA
<213>  Mycobacterium bovis

<400>  4
cgagagggga cggaaac                                                     17